(19) Europäisches Patentamt
European Patent Office
Office européen des brevets

(11) EP 0 987 005 A2

(12) # EUROPÄISCHE PATENTANMELDUNG

(43) Veröffentlichungstag:
**22.03.2000 Patentblatt 2000/12**

(21) Anmeldenummer: **99116873.3**

(22) Anmeldetag: **06.09.1999**

(51) Int. Cl.$^7$: **A61K 7/00**, A61K 7/42, A61K 7/48, A61K 7/32, A61K 9/10

(84) Benannte Vertragsstaaten:
**AT BE CH CY DE DK ES FI FR GB GR IE IT LI LU MC NL PT SE**
Benannte Erstreckungsstaaten:
**AL LT LV MK RO SI**

(30) Priorität: **18.09.1998 DE 19842732**

(71) Anmelder:
**Beiersdorf Aktiengesellschaft**
**20245 Hamburg (DE)**

(72) Erfinder:
- **Gers-Barlag, Heinrich, Dr.**
  **25495 Kummerfeld (DE)**
- **Müller, Anja**
  **23843 Rümpel (DE)**

(54) **Emulgatorfreie feindisperse Systeme vom Typ Öl-in-Wasser und Wasser-in-Öl**

(57) Pickering-Emulsionen, welche feindisperse Systeme vom Typ Wasser-in-Öl oder Öl-in-Wasser darstellen, enthaltend

(1) eine Ölphase, die mindestens ein Wachs und/oder ein Ölverdickungsmittel enthält,
(2) eine Wasserphase,
(3) mindestens einen Typ mikrofeiner Partikel, die

a) eine mittlere Partikelgröße von weniger als 200 nm haben, die
b) sowohl hydrophile als auch lipophile Eigenschaften zeigen, welche also amphiphilen Charakter besitzen und sowohl in Wasser als auch in Öl dispergierbar sind und die
c) gegebenenfalls oberflächlich beschichtet sind,

(4) mindestens einen Typ nicht-amphiphiler Metalloxidpigmente und
(5) höchstens 0,5 Gew.-% eines oder mehrerer Emulgatoren.

EP 0 987 005 A2

**Beschreibung**

Die vorliegende Erfindung betrifft emulgatorfreie feindisperse Systeme vom Typ Öl-in-Wasser und Wasser-in-Öl, bevorzugt als kosmetische oder dermatologische Zubereitungen, insbesondere als kosmetische oder dermatologische Lichtschutzzubereitungen.

Unter Emulsionen versteht man im allgemeinen heterogene Systeme, die aus zwei nicht oder nur begrenzt miteinander mischbaren Flüssigkeiten bestehen, die üblicherweise als Phasen bezeichnet werden. In einer Emulsion ist eine der beiden Flüssigkeiten in Form feinster Tröpfchen in der anderen Flüssigkeit dispergiert.

Sind die beiden Flüssigkeiten Wasser und Öl und liegen Öltröpfchen fein verteilt in Wasser vor, so handelt es sich um eine Öl-in-Wasser-Emulsion (O/W-Emulsion, z. B. Milch). Der Grundcharakter einer O/W-Emulsion ist durch das Wasser geprägt. Bei einer Wasser-in-Öl-Emulsion (W/O-Emulsion, z. B. Butter) handelt es sich um das umgekehrte Prinzip, wobei der Grundcharakter hier durch das Öl bestimmt wird.

Um die dauerhafte Dispergierung einer Flüssigkeit in einer anderen zu erreichen, ist bei Emulsionen im herkömmlichen Sinn der Zusatz einer grenzflächenaktiven Substanz (Emulgator) notwendig. Emulgatoren weisen einen amphiphilen Molekülaufbau auf, bestehend aus einem polaren (hydrophilen) und einem unpolaren (lipophilen) Molekülteil, die räumlich voneinander getrennt sind. In einfachen Emulsionen liegen in der einen Phase feindisperse, von einer Emulgatorhülle umschlossene Tröpfchen der zweiten Phase (Wassertröpfchen in W/O- oder Lipidvesikel in O/W-Emulsionen vor. Emulgatoren setzen die Grenzflächenspannung zwischen den Phasen herab, indem sie an der Grenzfläche zwischen beiden Flüssigkeiten angeordnet sind. Sie bilden an der Phasengrenze Öl/Wasser Grenzflächenfilme aus, wodurch dem irreversiblen Zusammenfließen der Tröpfchen entgegengewirkt wird. Zur Stabilisierung von Emulsionen werden häufig Emulgatorgemische verwendet.

Herkömmliche Emulgatoren können entsprechend ihrem hydrophilen Molekülteil in ionische (anionische, kationische und amphotere und nichtionische untergliedert werden:

- Das wohl bekannteste Beispiel eines anionischen Emulgators ist die Seife, als die man gewöhnlich die wasserlöslichen Natrium- oder Kaliumsalze der gesättigten und ungesättigten höheren Fettsäuren bezeichnet.
- Wichtige Vertreter der kationischen Emulgatoren sind die quartären Ammonium-Verbindungen.
- Der hydrophile Molekülteil nichtionischer Emulgatoren besteht häufig aus Glycerin, Polyglycerin, Sorbitanen, Kohlenhydraten bzw. Polyoxyethylenglykolen und ist meistens über Ester- und Etherbindungen mit dem lipophilen Molekülteil verknüpft. Dieser besteht üblicherweise aus Fettalkoholen, Fettsäuren oder Isofettsäuren.

Durch Variation der Struktur und der Größe des polaren und des unpolaren Molekülteils lassen sich Lipophilie und Hydrophilie von Emulgatoren in weiten Grenzen verändern.

Entscheidend für die Stabilität einer Emulsion ist die richtige Auswahl der Emulgatoren. Dabei sind die Charakteristiken aller im System enthaltenen Stoffe zu berücksichtigen. Betrachtet man z. B. Hautpflegeemulsionen, so führen polare Ölkomponenten und beispielsweise UV-Filter zu Instabilitäten. Neben den Emulgatoren werden daher noch andere Stabilisatoren verwendet, die beispielsweise die Viskosität der Emulsion erhöhen und/oder als Schutzkolloid wirken.

Emulsionen stellen einen wichtigen Produkttyp im Bereich kosmetischer und/oder dermatologischer Zubereitungen dar.

Kosmetische Zubereitungen werden im wesentlichen zur Hautpflege benutzt. Hautpflege im kosmetischen Sinn ist in erster Linie, daß die natürliche Funktion der Haut als Barriere gegen Umwelteinflüsse (z.B. Schmutz, Chemikalien, Mikroorganismen) und gegen den Verlust von körpereigenen Stoffen (z.B. Wasser, natürliche Fette, Elektrolyte) gestärkt oder wiederhergestellt wird. Wird diese Funktion gestört, kann es zu verstärkter Resorption toxischer oder allergener Stoffe oder zum Befall von Mikroorganismen und als Folge zu toxischen oder allergischen Hautreaktionen kommen.

Ziel der Hautpflege ist es ferner, den durch tägliches Waschen verursachten Fett- und Wasserverlust der Haut auszugleichen. Dies ist gerade dann wichtig, wenn das natürliche Regenerationsvermögen nicht ausreicht. Außerdem sollen Hautpflegeprodukte vor Umwelteinflüssen, insbesondere vor Sonne und Wind, schützen und die Hautalterung verzögern.

Medizinische topische Zusammensetzungen enthalten in der Regel ein oder mehrere Medikamente in wirksamer Konzentration. Der Einfachheit halber wird zur eindeutigen Unterscheidung zwischen kosmetischer und medizinischer Anwendung und entsprechenden Produkten auf die gesetzlichen Bestimmungen der Bundesrepublik Deutschland verwiesen (z.B. Kosmetikverordnung, Lebensmittel- und Arzneimittelgesetz).

An sich ist die Verwendung der üblichen Emulgatoren in kosmetischen oder dermatologischen Zubereitungen unbedenklich. Dennoch können Emulgatoren, wie letztlich jede chemische Substanz, im Einzelfalle allergische oder auf Überempfindlichkeit des Anwenders beruhende Reaktionen hervorrufen.

So ist beispielsweise bekannt, daß bestimmte Lichtdermatosen durch gewisse Emulgatoren, aber auch durch

verschiedene Fette und gleichzeitige Exposition von Sonnenlicht ausgelöst werden. Solche Lichtdermatosen werden auch "Mallorca-Akne" genannt. Es hat daher nicht an Versuchen gefehlt, die Menge an üblichen Emulgatoren auf ein Minimum, im Idealfall sogar vollständig zu reduzieren.

Eine Reduktion der benötigten Emulgatormenge kann z. B. erreicht werden, wenn ausgenutzt wird, daß feinstverteilte Feststoffteilchen eine zusätzlich stabilisierende Wirkung haben. Dabei kommt es zu einer Anreicherung des festen Stoffes an der Phasengrenze Öl/Wasser in Form einer Schicht, wodurch Zusammenfließen der dispersen Phasen verhindert wird. Von wesentlicher Bedeutung sind dabei nicht die chemischen, sondern die Oberflächeneigenschaften der Feststoffpartikel.

Pickering stellte um 1910 erstmals Paraffin-Wasser-Emulsionen her, die nur durch den Zusatz verschiedener Feststoffe wie basisches Kupfersulfat, basisches Eisensulfat oder andere Metallsulfate stabilisiert wurden. Diese Art von Emulsionen wird daher auch als Pickering-Emulsion bezeichnet.

Sozusagen die Urformen von Pickering-Emulsionen tauchten zunächst als unerwünschte Nebeneffekte bei unterschiedlichen technischen Prozessen auf, wie z. B. bei der sekundären Erdölförderung, der Extraktion von Bitumen aus Teersand und anderen Trennungsverfahren, an denen zwei nicht miteinander mischbare Flüssigkeiten und feine, dispergierte Feststoffpartikel beteiligt sind. Im allgemeinen handelt es sich hierbei um W/O-Emulsionen, die durch mineralische Feststoffe stabilisiert werden. Dementsprechend stand die Untersuchung entsprechender Systeme, wie z. B. der Systeme Öl-Wasser-Ruß oder Öl-Wasser-Schieferstaub zunächst im Mittelpunkt der Forschungsaktivitäten.

Grundlegende Untersuchungen haben dabei gezeigt, daß ein Charakteristikum für eine Pickering-Emulsion ist, daß die Feststoffpartikel an der Grenzfläche zwischen den beiden flüssigen Phasen angeordnet sind und dort sozusagen eine mechanische Barriere gegen die Vereinigung der Flüssigkeitströpfen bilden.

Eine relativ neue technische Entwicklung ist es, Pickering-Emulsionen als Grundlage für kosmetische oder dermatologische Zubereitungen zu verwenden.

Eine Möglichkeit, eine Feststoffstabilisierung im Sinne einer Pickering-Emulsion in einer kosmetischen oder dermatologischen Zubereitung vorzunehmen, ist nach May-Alert (*Pharmazie in unserer Zeit, 15. Jahrg. 1986, Nr. 1, 1-7)* beispielsweise, Emulgatorgemische zu verwenden, die sowohl anionische als auch kationische Tenside enthalten. Da beim Zusammengeben von Anion- und Kationtensiden immer unlösliche, elektroneutrale Verbindungen ausfallen, läßt sich durch gezieltes Ausfällen dieser neutralen Tenside in der Grenzfläche Öl/Wasser eine zusätzliche Feststoffstabilisierung erreichen.

Darüber hinaus beschreibt die Europäische Offenlegungsschrift 0 686 391 Emulsionen vom Typ Wasser-in-Öl, die frei von oberflächenaktiven Substanzen sind und nur durch Feststoffe stabilisiert werden. Zur Stabilisierung werden hier sphärische Polyalkylsilsesquioxan-Partikel eingesetzt, die einen Durchmesser von 100 nm bis zu 20 $\mu$m haben. Diese Emulsionen können nach dem oben gesagten als Pickering-Emulsionen bezeichnet werden.

Pickering-Emulsionen werden durch den Einsatz von geeigneten Feststoffen bzw. Pigmenten stabilisiert. Allerdings haben Pickering-Emulsionen des Standes der Technik im allgemeinen den Nachteil, daß sie gegen UV-Licht, insbesondere gegen UV-A-Licht nur unbefriedigend wirken, da Pigmente, die besonders zur Stabilisierung dieses Emulsionstyps geeignet sind, im allgemeinen eine unzureichende Lichtschutzwirkung haben.

Die schädigende Wirkung des ultravioletten Teils der Sonnenstrahlung auf die Haut ist allgemein bekannt. In Abhängigkeit von ihrer jeweiligen Wellenlänge haben die Strahlen verschiedene Wirkungen auf das Organ Haut: Die sogenannte UV-C-Strahlung mit einer Wellenlänge, die kleiner als 290 nm ist, wird von der Ozonschicht in der Erdatmosphäre absorbiert und hat daher keine physiologische Bedeutung. Dagegen verursachen Strahlen im Bereich zwischen 290 nm und 320 nm, dem sogenannten UV-B-Bereich, ein Erythem, einen einfachen Sonnenbrand oder sogar mehr oder weniger starke Verbrennungen. Als ein Maximum der Erythemwirksamkeit des Sonnenlichtes wird der engere Bereich um 308 nm angegeben.

Man hat lange Zeit fälschlicherweise angenommen, daß die langwellige UV-A-Strahlung mit einer Wellenlänge zwischen 320 nm und 400 nm nur eine vernachlässigbare biologische Wirkung aufweist und daß dementsprechend die UV-B-Strahlen für die meisten Lichtschäden an der menschlichen Haut verantwortlich seien. Inzwischen ist allerdings durch zahlreiche Studien belegt, daß UV-A-Strahlung im Hinblick auf die Auslösung photodynamischer, speziell phototoxischer Reaktionen und chronischer Veränderungen der Haut weitaus gefährlicher als UV-B-Strahlung ist. Auch kann der schädigende Einfluß der UV-B-Strahlung durch UV-A-Strahlung noch verstärkt werden.

Vorbeugender Schutz gegen UV-Strahlung, beispielsweise durch Auftrag von Lichtschutzfiltersubstanzen in Form einer kosmetischen oder dermatologischen Formulierung auf die Haut, ist daher von grundsätzlicher Wichtigkeit. Dies gilt insbesondere auch für Formulierungen, deren hauptsächlicher Zweck nicht der Schutz vor Sonnenlicht ist, die aber im allgemeinen auf Körperpartien aufgetragen werden, welche üblicherweise dem Tageslicht ausgesetzt sind, wie beispielsweise Gesichts- und Körperpflegezubereitungen.

Aufgabe war daher, den Nachteilen des Standes der Technik Abhilfe zu schaffen. Insbesondere sollten kosmetische und dermatologische Grundlagen für kosmetische und dermatologische Zubereitungen zur Verfügung gestellt werden, die sich durch gute Hautverträglichkeit und eine hohe UV-Schutzleistung, insbesondere eine hohe UV-A-Schutzleistung auszeichnen.

Ferner war eine Aufgabe der vorliegenden Erfindung, Produkte mit einer möglichst breiten Anwendungsvielfalt zur Verfügung zu stellen. Beispielsweise sollten Grundlagen für Zubereitungsformen wie Reinigungsemulsionen, Gesichts- und Körperpflegezubereitungen, aber auch ausgesprochen medizinisch-pharmazeutische Darreichungsformen geschaffen werden, zum Beispiel Zubereitungen gegen Akne und andere Hauterscheinungen.

Es war erstaunlich und für den Fachmann in keiner Weise vorauszusehen, daß Pickering-Emulsionen, welche feindisperse Systeme vom Typ Wasser-in-Öl oder Öl-in-Wasser darstellen, enthaltend

(1) eine Ölphase,
(2) eine Wasserphase,
(3) mindestens einen Typ mikrofeiner Partikel, die

a) eine mittlere Partikelgröße von weniger als 200 nm haben, die
b) sowohl hydrophile als auch lipophile Eigenschaften zeigen, welche also amphiphilen Charakter besitzen und sowohl in Wasser als auch in Öl dispergierbar sind und die
c) gegebenenfalls oberflächlich beschichtet sind,

(4) mindestens einen Typ nicht-amphiphiler Metalloxidpigmente und
(5) höchstens 0,5 Gew.-% eines oder mehrerer Emulgatoren den Nachteilen des Standes der Technik abhelfen.

Es ist erfindungsgemäß besonders vorteilhaft, wenn die Zubereitungen deutlich weniger als 0,5 Gew.-% eines oder mehrerer Emulgatoren enthalten bzw. sogar gänzlich emulgatorfrei sind.

Die erfindungsgemäßen Zubereitungen sind in jeglicher Hinsicht überaus befriedigende Präparate, die insbesondere geeignet sind, um als Grundlage für Zubereitungsformen mit vielfältigen Anwendungszwecken zu dienen. Zudem zeichnen sich die erfindungsgemäßen Zubereitungen durch eine ausgezeichnete Hautverträglichkeit aus.

Ferner war erstaunlich, daß erfindungsgemäße Zubereitungen eine höhere Wirksamkeit gegen UV-Strahlung, insbesondere eine höhere UV-A-Schutzleistung zeigen als übliche Sonnenschutzformulierungen auf Basis von Pickering-Emulsionen.

Zwar kennt der Stand der Technik neben Pickering-Emulsionen emulgatorfreie, feindisperse kosmetische oder dermatologische Zubereitungen, die im allgemeinen als Hydrodispersionen bezeichnet werden und welche Dispersionen einer flüssigen, halbfesten oder festen inneren (diskontinuierlichen) Lipidphase in einer äußeren wäßrigen (kontinuierlichen) Phase darstellen. Allerdings konnte der Stand der Technik nicht den Weg zur vorliegenden Erfindung weisen.

Bei Hydrodispersionen einer flüssigen Lipidphase in einer äußeren wäßrigen Phase kann die Stabilität beispielsweise dadurch gewährleistet werden, daß in der wäßrigen Phase ein Gelgerüst aufgebaut wird, in welchem die Lipidtröpfchen stabil suspendiert sind.

Die Deutsche Offenlegungsschrift 44 25 268 beschreibt stabile feindisperse, emulgatorfreie kosmetische oder dermatologische Zubereitungen vom Typ Öl-in-Wasser, die neben einer Öl- und einer Wasserphase einen oder mehrere Verdicker aus der Gruppe der Acrylsäurepolymere, Polysaccharide und deren Alkylether enthalten, wobei für diese Verdicker eine Grenzflächenspannungserniedrigung nicht meßbar sein darf.

Basierend auf ähnlichen Hydrodispersionen werden in der Deutschen Offenlegungsschrift 43 03 983 kosmetische oder dermatologische Lichtschutzformulierungen offenbart, die im wesentlichen frei von Emulgatoren sind, wobei in die Lipidphase der Hydrodispersion anorganische Mikropigmente eingearbeitet sind, die als UV-Filtersubstanzen dienen.

O/W-Pickering-Emulsionen im Sinne der vorliegenden Erfindung hingegen sind erhältlich, indem man erfindungsgemäße, für die Herstellung von O/W-Pickering-Emulsionen geeignete amphiphile Partikel zunächst <u>in der Wasserphase</u> dispergiert und die Wasserphase anschließend mit der Fettphase vereinigt. Erfindungsgemäße W/O-Pickering-Emulsionen sind dagegen durch Dispergieren von erfindungsgemäßen, für die Herstellung von W/O-Pickering-Emulsionen geeigneten amphiphilen Partikeln <u>in der Fettphase</u> erhältlich.

Vorteilhaft im Sinne der vorliegenden Erfindung werden die nicht-amphiphilen Metalloxidpigmente gewählt aus der Gruppe der Oxide des Titans ($TiO_2$), Zinks (ZnO), Eisens (z.B. $Fe_2O_3$), Zirkoniums ($ZrO_2$), Siliciums ($SiO_2$), Mangans (z.B. MnO), Aluminiums ($Al_2O_3$), Cers (z.B. $Ce_2O_3$), Mischoxiden der entsprechenden Metalle sowie Abmischungen aus solchen Oxiden. Besonders bevorzugt handelt es sich um nicht-amphiphile Pigmente auf der Basis von $TiO_2$.

Die nicht-amphiphilen Pigmente liegen erfindungsgemäß vorteilhaft in hydrophober Form vor, d. h., daß sie oberflächlich wasserabweisend behandelt sind. Diese Oberflächenbehandlung kann darin bestehen, daß die Pigmente nach an sich bekannten Verfahren mit einer dünnen hydrophoben Schicht versehen werden. Vorteilhafte hydrophobe $TiO_2$-Pigmente sind beispielsweise unter der Handelsbezeichnung T 805 von der Firma Degussa erhältlichen (CAS-Nr. 100209-12-9, ex 13463-67-7).

Eine weitere vorteilhafte hydrophobe Beschichtung besteht aus Aluminiumhydroxid bzw. Aluminiumoxidhydrat

(auch: Alumina, CAS-Nr.: 1333-84-2), auf das anschließend Stearinsäure aufgebracht wird. Es ist darüber hinaus auch bevorzugt, andere hydrophobe Beschichtungen auf mit Alumina vorbehandelte Metalloxidpartikel aufzubringen, wie z. B. Polyorganosiloxane. Vorteilhafte mit Alumina und Stearinsäure beschichtete, nichtamphiphile Titandioxidpigmente sind beispielsweise unter der Handelsbezeichnung UV Titan M160 bei Kemira erhältlich.

Erfindungsgemäß vorteilhaft sind ferner Dispersionen von ultrafeinem Titandioxid in Ölen bzw. ölige Titandioxid-Suspensionen, z. B. Titandioxid in Caprylic-/Capric Triglycerid, einem Gemisch von Triglyceriden hauptsächlich der Caprylsäure [$CH_3(CH_2)_6COOH$] und der Caprinsäure [$CH_3(CH_2)_8COOH$]. Bevorzugt sind beispielsweise die unter der Handelsbezeichnung Tioveil TG bei der Firma Solaveil erhältlichen öligen Titandioxid-Suspensionen.

Die nicht-amphiphilen Metalloxidpigmente werden im Sinne der vorliegenden Erfindung ferner bevorzugt aus der Gruppe der hydrophilen bzw. hydrophil beschichteten Pigmente gewählt. Die Oberflächenbehandlung kann darin bestehen, daß die Pigmente nach an sich bekannten Verfahren mit einer dünnen hydrophilen Schicht versehen werden.

Weiter vorteilhaft sind beispielsweise wäßrige Dispersionen von hydrophilen Titandioxidpigmenten, die auch weitere, z. B. konservierende Bestandteile enthalten können, wie beispielsweise Propylenglykol. Bevorzugt sind z. B. die unter der Handelsbezeichnung Tioveil AQ bei der Firma Solaveil erhältlichen wäßrigen TitandioxidSuspensionen.

Die Gesamtmenge an nicht-amphiphilen Metalloxidpigmenten, insbesondere hydrophoben Mikropigmenten in den fertigen kosmetischen oder dermatologischen Zubereitungen wird vorteilhaft kleiner als 20 Gew.-%, bevorzugt zwischen 0,5 und 10,0 Gew.-% gewählt, bezogen auf das Gesamtgewicht der Zubereitungen.

Es ist ferner vorteilhaft, wenngleich nicht zwingend, wenn die erfindungsgemäßen Pickering -Emulsionen Hilfsstoffe enthalten, die die Stabilität dieser Zubereitungen zusätzlich erhöhen können, beispielsweise Stoffe (einer oder mehrere), die gewählt werden aus der Gruppe der Wachse und/oder Ölverdickungsmittel, der Hydrokolloide sowie der Elektrolyte.

Es ist ferner vorteilhaft, wenn die erfindungsgemäßen Pickering-Emulsionen Hilfsstoffe enthalten, die zur Verminderung oder Verhinderung eines stumpfen oder trockenen Hautgefühls nach deren Auftragen beitragen können, wobei der hauptsächliche Zweck dieser Stoffe ein anderer sein kann. Vorzugsweise werden diese Stoffe beispielsweise gewählt aus der Gruppe der unsymmetrisch substituierten s-Triazinderivate, der Cyclodextrine, der Filmbildner und der polymeren Moisturizer, wobei diese Stoffe sowohl einzeln als auch im Gemisch vorliegen können.

Die kosmetischen Eigenschaften der erfindungsgemäßen Pickering-Emulsionen können zusätzlich beispielsweise noch dadurch verbessert werden, daß in der Ölphase auch Öle eingesetzt werden, welche eine Viskosität haben, die kleiner als 30 mPa • s, insbesondere kleiner als 20 mPa • s ist (bestimmt mit einem Rheometer der Firma Contraves (Rheomat 108E) bei einem Schergefälle von 500/s und einer Temperatur von 25 °C).

<u>**Mikrofeine amphiphile Partikel:**</u>

Der amphiphile Charakter der erfindungsgemäßen mikrofeinen Partikel zeigt sich beispielsweise darin, daß diese sowohl in Wasser als auch in Öl dispergierbar sind.

Es ist vorteilhaft, den mittleren Partikeldurchmesser der amphiphilen Partikel zwischen 1 nm und 200 nm, besonders vorteilhaft zwischen 5 nm und 100 nm zu wählen.

Es ist ferner vorteilhaft, die Konzentration aller erfindungsgemäßen amphiphilen Partikel größer als 0,1 Gew.-%, besonders vorteilhaft zwischen 0,1 Gew.-% und 30 Gew.-%, bezogen auf das Gesamtgewicht der Zubereitungen, zu wählen.

Vorteilhaft im Sinne der vorliegenden Erfindung sind alle amphiphilen Partikel, die geeignet sind, Pickering-W/O-Emulsionen bzw. Pickering-O/W-Emulsionen zu stabilisieren. Im wesentlichen unerheblich für die vorliegende Erfindung ist es, in welcher der gegebenenfalls natürlich vorkommenden Modifikationen die Partikel vorliegen.

Vorzugsweise werden zur Stabilisierung der Pickering-Emulsionen unbehandelte, nahezu reine Pigmentpartikel verwendet, z. B. solche, welche auch als Farbstoff in der Lebensmittelindustrie verwendet werden können. Vorteilhaft sind beispielsweise die bei der Firma Merck erhältlichen Zinkoxid-Pigmente sowie solche, die unter den Handelsbezeichnungen Zinkoxid neutral bei Haarmann & Reimer oder NanoX von der Harcros Chemical Group erhältlich sind.

Pickering-Emulsionen im Sinne der vorliegenden Erfindung werden ebenfalls vorteilhaft durch anorganische Pigmente stabilisiert, die oberflächlich wasserabweisend behandelt („gecoatet") sind, wobei gleichzeitig der amphiphile Charakter gebildet werden bzw. erhalten bleiben soll. Diese Oberflächenbehandlung kann z. B. darin bestehen, daß die Pigmente nach an sich bekannten Verfahren partiell mit einer dünnen hydrophoben Schicht oder aber nahezu vollständig mit einer amphiphilen Schicht versehen werden.

Ein solches Verfahren, das im folgenden am Beispiel von Titandioxid beschrieben wird, besteht beispielsweise darin, daß die hydrophobe Oberflächenschicht nach einer Reaktion gemäß

$$n\ TiO_2 + m\ (RO)_3Si\text{-}R' \rightarrow n\ TiO_2\ (\text{oberfl.})$$

erzeugt wird. n und m sind dabei nach Belieben einzusetzende stöchiometrische Parameter, R und R' die gewünschten organischen Reste. Besonders vorteilhaft sind $TiO_2$-Pigmente, beispielsweise die mit Aluminiumstearat beschichteten, unter der Handelsbezeichnung MT 100 T bei der Firma TAYCA erhältlichen.

Eine weitere vorteilhafte Beschichtung der anorganische Pigmente besteht aus Dimethylpolysiloxan (auch: Dimethicon), einem Gemisch vollmethylierter, linearer Siloxanpolymere, die endständig mit Trimethylsiloxy-Einheiten blockiert sind. Besonders vorteilhaft im Sinne der vorliegenden Erfindung sind Zinkoxid-Pigmente, die auf diese Weise beschichtet werden.

Vorteilhaft ist ferner eine Beschichtung der amphiphilen Pigmente mit einem Gemisch aus Dimethylpolysiloxan, insbesondere Dimethylpolysiloxan mit einer durchschnittlichen Kettenlänge von 200 bis 350 Dimethylsiloxan-Einheiten, und Silicagel, welches auch als Simethicone bezeichnet wird. Es ist insbesondere von Vorteil, wenn die amphiphilen Pigmente zusätzlich mit Aluminiumhydroxid bzw. Aluminiumoxidhydrat (auch: Alumina, CAS-Nr.: 1333-84-2) beschichtet sind. Besonders vorteilhaft sind Titandioxide, die mit Simethicone und Alumina beschichtet sind, wobei die Beschichtung auch Wasser enthalten kann. Ein Beispiel hierfür ist das unter dem Handelsnamen Eusolex T2000 bei der Firma Merck erhältliche Titandioxid.

Vorteilhaft im Sinne der vorliegenden Erfindung ist ferner die Verwendung einer Mischung verschiedener Pigmenttypen sowohl innerhalb eines Kristalls, beispielsweise als Eisenmischoxid, als auch durch Kombination mehrerer Pigmenttypen innerhalb einer Zubereitung.

Vorzugsweise werden die erfindungsgemäßen Pickering-Emulsionen ferner durch Bornitridpartikel stabilisiert, beispielsweise durch die im folgenden aufgelisteten Bornitride:

| Handelsname | erhältlich bei |
|---|---|
| Boron Nitride Powder | Advanced Ceramics |
| Boron Nitride Powder | Sintec Keramik |
| Ceram Blanche | Kawasaki |
| HCST Boron Nitride | Stark |
| Très BN® | Carborundum |
| Wacker-Bornitrid BNP | Wacker-Chemie |

Vorteilhaft ist es, den mittleren Partikeldurchmesser der verwendeten Bornitridpartikel kleiner als 20 µm, besonders vorteilhaft kleiner als 15 µm zu wählen.

Pickering-Emulsionen im Sinne der vorliegenden Erfindung werden ebenfalls vorteilhaft durch Bornitridpartikel stabilisiert, die oberflächlich wasserabweisend behandelt („gecoatet") sind, wobei gleichzeitig der amphiphile Charakter gebildet werden bzw. erhaften bleiben soll.

Eine vorteilhafte Beschichtung der Bornitridpartikel besteht aus Dimethylpolysiloxan (Dimethicon). Vorteilhaft sind beispielsweise die bei der Firma Carborundum unter der Handelsbezeichnung Très BN® UHP 1106 erhältlichen, mit Dimethicon behandelten Bornitridpartikel.

Vorteilhaft ist ferner eine Beschichtung der Bornitridpartikel mit Polymethylhydrogensiloxan, einem linearen Polysiloxan, welches auch als Methicone bezeichnet wird. Vorteilhafte, mit Methicone behandelte Bornitridpartikel sind beispielsweise die bei der Firma Carborundum unter der Handelsbezeichnung Très BN® UHP 1107 erhältlichen.

Es ist ferner vorteilhaft, die erfindungsgemäßen Pickering-Emulsionen durch mikrofeine Polymerpartikel zu stabilisieren.

Vorteilhafte mikrofeine Polymerpartikel sind im Sinne der vorliegenden Erfindung beispielsweise Polycarbonate, Polyether, Polyethylen, Polypropylen, Polyvinylchlorid, Polystyrol, Polyamide, Polyacrylate und dergleichen mehr.

Erfindungsgemäß vorteilhaft sind beispielsweise mikrofeine Polyamid-Partikel, welche unter der Handelsbezeichnung SP-500 bei der Firma TORAY erhältlich sind. Ferner vorteilhaft sind Polyamid 6- (auch: Nylon 6) bzw. Polyamid 12- (auch: Nylon 12) Partikel. Polyamid 6 ist das aus ε-Aminocapronsäure (6-Aminohexansäure) oder ε-Caprolactam aufgebaute Polyamid [Poly(ε-caprolactam)], und Polyamid 12 ist ein Poly(ε-laurinlactam) aus ε-Laurinlactam. Vorteilhaft im Sinne der vorliegenden Erfindung sind beispielsweise Orgasol® 1002 (Polyamid 6) und Orgasol® 2002 (Polyamid 12) von der Firma ELF ATOCHEM.

Weitere vorteilhafte Polymerpartikel sind mikrofeine Polymethacrylate. Solche Partikel sind beispielsweise unter der Handelsbezeichnung POLYTRAP® bei der Firma DOW CHEMICAL erhältlich.

Es ist insbesondere vorteilhaft, wenngleich nicht zwingend, wenn die verwendeten mikrofeinen Polymerpartikel oberflächlich beschichtet sind. Diese Oberflächenbehandlung kann darin bestehen, daß die Pigmente nach an sich bekannten Verfahren mit einer dünnen hydrophilen Schicht versehen werden. Vorteilhafte Beschichtungen bestehen beispielsweise aus $TiO_2$, $ZrO_2$ oder auch weiteren Polymeren, wie beispielsweise Polymethylmethacrylat.

Besonders vorteilhafte mikrofeine Polymerpartikel im Sinne der vorliegenden Erfindung sind ferner nach dem in der US-Patentschrift 4,898,913 beschriebenen Verfahren zur hydrophilen Beschichtung hydrophober Polymerpartikel erhältlich.

Vorteilhaft in ist es, den mittleren Partikeldurchmesser der verwendeten mikrofeinen Polymerpartikel kleiner als 100 µm, besonders vorteilhaft kleiner als 50 µm zu wählen. Dabei ist es im wesentlichen unerheblich, in welcher Form (Plättchen, Stäbchen, Kügelchen etc.) die verwendeten Polymerpartikel vorliegen.

Desweiteren ist es vorteilhaft, die erfindungsgemäßen Pickering-Emulsionen durch modifizierte Polysaccharide zu stabilisieren.

Modifizierte Polysaccharide im Sinne der vorliegenden Erfindung sind beispielsweise durch Umsetzung von Stärke mit mono-, bi- oder polyfunktionellen Reagenzien bzw. Oxidations-Mitteln in weitgehend polymeranalog verlaufenden Reaktionen erhältlich.

Solche Reaktionen basieren im wesentlichen auf Umwandlungen der Hydroxy-Gruppen der Polyglucane durch Veretherung, Veresterung oder selektive Oxidation. Dabei entstehen z. B. sogenannte Stärkeether und Stärkeester der allgemeinen Strukturformel

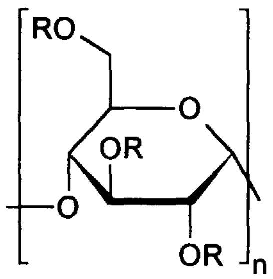

**Strukturformel (I)**

worin R beispielsweise ein Wasserstoff und/oder einen Alkyl- und/oder Aralkylrest (im Fall der Stärkeether) oder ein Wasserstoff und/oder einen organischen und/oder anorganischen Säure-Rest (im Fall der Stärkeester) darstellen kann. Stärkeether und Stärkeester sind vorteilhafte modifizierte Polysaccharide im Sinne der vorliegenden Erfindung.

Besonders vorteilhafte Stärkeether sind z. B. solche, die durch Veretherung von Stärke mit Tetramethylolacetylendihamstoff erhältlich sind und welche als Amylum non mucilaginosum (nicht quellende Stärke) bezeichnet werden.

Insbesondere vorteilhaft sind ferner Stärkeester und deren Salze, beispielsweise die Natrium- und/oder Aluminiumsalze niedrigsubstituierter Halbester der Stärke, insbesondere Natrium Stärke n-Octenylsuccinat der Strukturformel (I), worin R sich durch die folgende Struktur auszeichnet

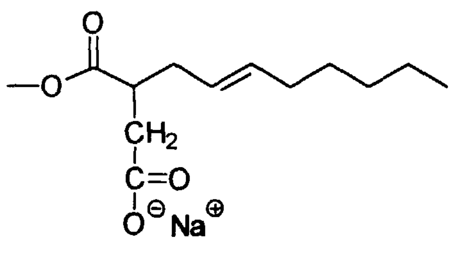

und welches z. B. unter der Handeisbezeichnung Amiogum® 23 bei der Firma CERESTAR erhältlich ist sowie Aluminium Stärke Octenylsuccinate, insbesondere die unter den Handelsbezeichnungen Dry Flo® Elite LL und Dry Flo® PC bei der Firma CERESTAR erhältlichen.

Vorteilhaft ist es, den mittleren Partikeldurchmesser der verwendeten modifizierten Polysaccharide kleiner als 20 µm, besonders vorteilhaft kleiner als 15 µm zu wählen.

Die Liste der genannten modifizierten Polysaccharide, die erfindungsgemäße PickeringEmulsionen stabilisieren können, soll selbstverständlich nicht limitierend sein. Modifizierte Polysaccharide im Sinne der vorliegenden Erfindung sind auf zahlreichen, an sich bekannten Wegen, sowohl chemischer als auch physikalischer Natur erhältlich.

Die vorstehend genannten amphiphilen Partikel eignen sich hervorragend sowohl zur Stabilisierung von W/O-Pickering-Emulsionen als auch zur Stabilisierung von O/W-Pickering -Emulsionen. Nachfolgend werden erfindungsgemäße mikrofeine Partikel genannt, welche vorteilhaft insbesondere einen der beiden Emulsionstypen W/O bzw. O/W stabilisieren.

W/O-Pickering-Emulsionen:

Der Wasserphasenanteil der erfindungsgemäßen W/O-Pickering-Emulsionen wird vorzugsweise aus dem Bereich von 0,5 bis 75 Gew. % gewählt, bezogen auf das Gesamtgewicht der Formulierungen.

Vorteilhaft zur Stabilisierung von W/O-Pickering-Emulsionen sind insbesondere auch Magnesiumsilicate (auch: Talkum), beispielsweise die unter der Handelsbezeichnung Talkum Micron bei der Firma Grolmann erhältlichen.

O/W-Pickering-Emulsionen:

Der Fettphasenanteil der erfindungsgemäßen O/W-Pickering-Emulsionen wird vorzugsweise aus dem Bereich von 0,5 bis 75 Gew. % gewählt, bezogen auf das Gesamtgewicht der Formulierungen.

Besonders vorteilhaft zur Stabilisierung von O/W-Pickering-Emulsionen sind im Sinne der vorliegenden Erfindung auch unbehandelte, nahezu reine Pigmentpartikel, beispielsweise Titandioxid-Pigmente, insbesondere solche, die unter der Handelsbezeichnung KRONOS® 1171 ($TiO_2$) von der Firma Kronos Titan erhältlich sind.

O/W-Pickering-Emulsionen werden im Sinne der vorliegenden Erfindung ferner besonders vorteilhaft durch amphiphile Metalloxidpartikel stabilisiert, die mit Aluminiumhydroxid und/oder Siliziumdioxid überzogen („gecoatet") sind. Vorteilhafte Ausführungsformen sind beispielsweise Titandioxidpartikel, die unter dem Namen EUSOLEX® TA bei der Firma Merck erhältlich sind.

Es ist ferner vorteilhaft, wenngleich nicht zwingend, die erfindungsgemäßen amphiphilen Partikel mit weiteren Partikeln zu kombinieren, welche gegebenenfalls auch zur Stabilisierung der Pickering-Emulsionen beitragen können.

Solche Partikel sind beispielsweise Titandioxidpigmente, die mit Octylsilanol beschichtet sind, und/oder Siliciumdioxidpartikel, die oberflächlich wasserabweisend behandelt sind. Geeignete Siliciumdioxidpartikel sind beispielsweise sphärische PolyalkylsilsesquioxanPartikel, wie sie in der Europäischen Offenlegungsschrift 0 686 391 erwähnt werden. Solche Polyalkylsilsesquioxan-Partikel sind beispielsweise unter den Handelsbezeichnungen Aerosil R972 und Aerosil 200V bei der Firma Degussa erhältlich. Geeignete Titandioxidpartikel sind unter der Handelsbezeichnung T805 ebenfalls bei der Firma Degussa erhältlich.

Die erfindungsgemäßen Pickering-Emulsionen können als Grundlage für kosmetische oder dermatologische Formulierungen dienen. Diese können wie üblich zusammengesetzt sein und beispielsweise zur Behandlung und der Pflege der Haut, als Lippenpflegeprodukt, als Schmink- bzw. Abschminkprodukt in der dekorativen Kosmetik oder als Lichtschutzpräparat dienen. Zur Anwendung werden die erfindungsgemäßen kosmetischen und dermatologischen Zubereitungen in der für Kosmetika üblichen Weise auf die Haut in ausreichender Menge aufgebracht.

Entsprechend können kosmetische oder topische dermatologische Zusammensetzungen im Sinne der vorliegenden Erfindung, je nach ihrem Aufbau, beispielsweise verwendet werden als Hautschutzcrème, Reinigungsmilch, Sonnenschutzlotion, Nährcrème, Tages- oder Nachtcrème usw. Es ist gegebenenfalls möglich und vorteilhaft, die erfindungsgemäßen Zusammensetzungen als Grundlage für pharmazeutische Formulierungen zu verwenden.

Die erfindungsgemäßen kosmetischen und dermatologischen Zubereitungen können kosmetische Hilfsstoffe enthalten, wie sie üblicherweise in solchen Zubereitungen verwendet werden, z.B. Konservierungsmittel, Bakterizide, Parfüme, Substanzen zum Verhindern des Schäumens, Farbstoffe, Pigmente, die eine färbende Wirkung haben, Verdickungsmittel, weichmachende, anfeuchtende und/oder feuchthaltende Substanzen, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösemittel oder Silikonderivate.

Erfindungsgemäße Pickering-Emulsionen können auch Verdickungsmittel enthalten, um die taktilen Eigenschaften der Emulsion zu verbessem.

Insbesondere können die erfindungsgemäßen Pickering-Emulsionen auch Antioxidantien enthalten. Erfindungsgemäß können als günstige Antioxidantien alle für kosmetische und/oder dermatologische Anwendungen geeigneten oder gebräuchlichen Antioxidantien verwendet werden.

Vorteilhaft werden die Antioxidantien gewählt aus der Gruppe bestehend aus Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. $\alpha$-Carotin, $\beta$-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, $\gamma$-Linoleyl-, Chole-

steryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis μmol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-A-palmitat) sowie Koniferylbenzoat des Benzoeharzes, Rutinsäure und deren Derivate, α-Glycosylrutin, Ferulasäure, Furfurylidenglucitol, Camosin, Butylhydroxytoluol, Butylhydroxyanisol, Nordihydroguajakharzsäure, Nordihydroguajaretsäure, Trihydroxybutyrophenon, Hamsäure und deren Derivate, Mannose und deren Derivate, Zink und dessen Derivate (z.B. ZnO, $ZnSO_4$) Selen und dessen Derivate (z.B. Selenmethionin), Stilbene und deren Derivate (z.B. Stilbenoxid, Trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Die Menge der vorgenannten Antioxidantien (eine oder mehrere Verbindungen) in den erfindungsgemäßen Zubereitungen beträgt vorzugsweise 0,001 bis 30 Gew.-%, besonders bevorzugt 0,05 - 20 Gew.-%, insbesondere 1 - 10 Gew.-%, bezogen auf das Gesamtgewicht der Zubereitung.

Sofern Vitamin E und/oder dessen Derivate das oder die Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.

Sofern Vitamin A, bzw. Vitamin-A-Derivate, bzw. Carotine bzw. deren Derivate das oder die Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.

Günstig sind auch kosmetische und dermatologische Zubereitungen, die in der Form eines Sonnenschutzmittels vorliegen. Vorzugsweise enthalten diese mindestens eine UV-A-Filtersubstanz und/oder mindestens eine UV-B-Filtersubstanz.

Es ist aber auch vorteilhaft im Sinne der vorliegenden Erfindungen, solche kosmetischen und dermatologischen Zubereitungen zu erstellen, deren hauptsächlicher Zweck nicht der Schutz vor Sonnenlicht ist, die aber dennoch einen zusätzlichen Gehalt an UV-Schutzsubstanzen enthalten. So werden beispielsweise in Tagescrèmes gewöhnlich UV-A- bzw. UV-B-Filtersubstanzen eingearbeitet.

Vorteilhaft können erfindungsgemäße Zubereitungen weitere Substanzen enthalten, die UV-Strahlung im UV-B-Bereich absorbieren, wobei die Gesamtmenge der Filtersubstanzen z. B. 0,1 Gew.-% bis 30 Gew.-%, vorzugsweise 0,5 bis 10 Gew.-%, insbesondere 1,0 bis 6,0 Gew.-% beträgt, bezogen auf das Gesamtgewicht der Zubereitungen, um kosmetische Zubereitungen zur Verfügung zu stellen, die das Haar bzw. die Haut vor dem gesamten Bereich der ultravioletten Strahlung schützen.

Enthalten die erfindungsgemäßen Emulsionen UV-B-Filtersubstanzen, können diese öllöslich oder wasserlöslich sein. Erfindungsgemäß vorteilhafte öllösliche UV-B-Filter sind z.B.:

- 3-Benzylidencampher-Derivate, vorzugsweise 3-(4-Methylbenzyliden)campher, 3-Benzylidencampher;
- 4-Aminobenzoësäure-Derivate, vorzugsweise 4-(Dimethylamino)-benzoësäure(2-ethylhexyl)ester, 4-(Dimethylamino)benzoësäureamylester;
- Ester der Zimtsäure, vorzugsweise 4-Methoxyzimtsäure(2-ethylhexyl)ester, 4-Methoxyzimtsäureisopentylester;
- Ester der Salicylsäure, vorzugsweise Salicylsäure(2-ethylhexyl)ester, Salicylsäure(4-isopropylbenzyl)ester, Salicylsäurehomomenthylester,
- Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
- Ester der Benzalmalonsäure, vorzugsweise 4-Methoxybenzalmalonsäuredi(2-ethylhexyl)ester,
- hinsichtlich der $C_3$-Achse des Triazingrundkörpers symmetrisch Triazinderivate, vorzugsweise 4,4',4''-(1,3,5-Triazin-2,4,6-triyltriimino)-tris-benzoesäure-tris(2-ethylhexylester),
- Benzotriazolderivate, vorzugsweise 2,2'-Methylen-bis-(6-(2H-benzotriazol-2-yl)-4-(1,1,3,3-tetramethylbutyl)-phenol)
- sowie an Polymere gebundene UV-Filter.

Vorteilhafte wasserlösliche UV-B-Filter sind z.B.:

- Salze der 2-Phenylbenzimidazol-5-sulfonsäure wie ihr Natrium-, Kalium- oder ihr Triethanolammonium-Salz, sowie die Sulfonsäure selbst;
- Sulfonsäure-Derivate von Benzophenonen, vorzugsweise 2-Hydroxy-4-methoxy-benzophenon-5-sulfonsäure und

ihre Salze;

- Sulfonsäure-Derivate des 3-Benzylidencamphers, wie z.B. 4-(2-Oxo-3-bornylidenmethyl)benzolsulfonsäure, 2-Methyl-5-(2-oxo-3-bornylidenmethyl)sulfonsäure und ihre Salze.

Die Liste der genannten UV-B-Filter, die in den erfindungsgemäßen Pickering-Emulsionen verwendet werden können, soll selbstverständlich nicht limitierend sein.

Es kann auch von Vorteil sein, in erfindungsgemäßen Pickering-Emulsionen weitere UV-A-Filter zu verwenden, die bisher üblicherweise in kosmetischen Zubereitungen enthalten sind. Bei diesen Substanzen handelt es sich vorzugsweise um Derivate des Dibenzoylmethans, insbesondere um 1-(4'-tert.Butylphenyl)-3-(4'-methoxyphenyl)propan-1,3-dion und um 1-Phenyl-3-(4'-isopropylphenyl)propan-1,3-dion.

Weitere vorteilhafte UV-A-Filtersubstanzen sind die Phenylen-1,4-bis-(2-benzimidazyl)-3,3'-5,5'-tetrasulfonsäure:

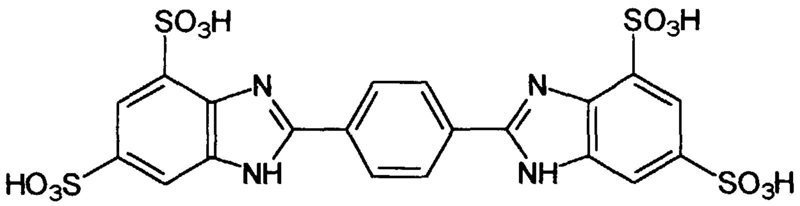

und ihre Salze, besonders die entsprechenden Natrium-, Kalium- oder Triethanolammonium-Salze, insbesondere das Phenylen-1,4-bis-(2-benzimidazyl)-3,3'-5,5'-tetrasulfonsäure-bis-natriumsalz:

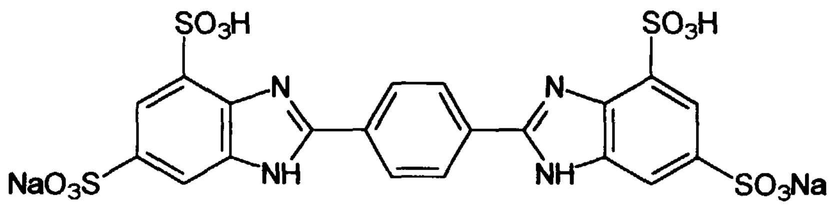

sowie das 1,4-Di(2-oxo-10-sulfo-3-bornylidenmethyl)-Benzol und dessen Salze (besonders die entprechenden 10-Sulfato-verbindungen, insbesondere das entsprechende Natrium-, Kalium- oder Triethanolammonium-Salz), das auch als Benzol-1,4-di(2-oxo-3-bornylidenmethyl-10-sulfonsäure) bezeichnet wird und sich durch die folgende Struktur auszeichnet:

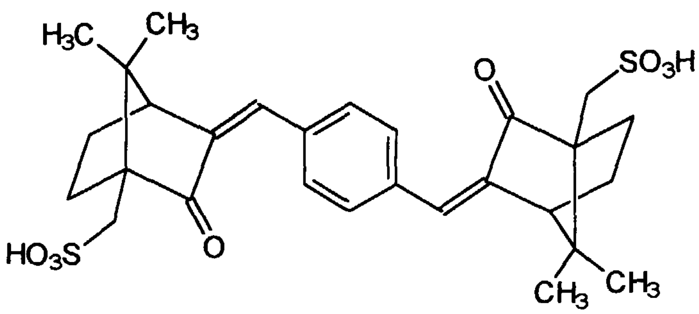

Auch Zubereitungen, die weitere UV-A-Filter enthalten, sind Gegenstand der Erfindung. Es können die für die UV-B-Kombination verwendeten Mengen eingesetzt werden.

Die nachfolgenden Beispiele sollen die vorliegende Erfindung verdeutlichen, ohne sie einzuschränken. Die Zahlenwerte in den Beispielen bedeuten Gewichtsprozente, bezogen auf das Gesamtgewicht der jeweiligen Zubereitungen.

Beispiele:

| | 1 | 2 | 3 | 4 | 5 | 6 |
|---|---|---|---|---|---|---|
| | W/O | W/O | W/O | O/W | O/W | O/W |
| amphiphiles Titandioxid (Eusolex T2000) | 2 | 4 | 6 | 3 | 5 | 2 |
| amphiphiles Zinkoxid | 5 | | 4 | | | 4 |
| nicht-amphiphiles Titandioxid (Titandioxid T805) | | | 2 | | 2 | |
| nicht-amphiphiles Titandioxid (Sperse® TN) | 5 | | | | | 2 |
| nicht-amphiphiles Titandioxid (UV Titan M160) | | 2 | | | | |
| nicht-amphiphiles Titandioxid (Tioveil TG) | | | | 2 | | |
| Silica (Aerosil R972) | | 1 | 0,5 | | | |
| Talkum (Talkum Micron) | | 0,5 | | | | |
| Bornitrid | | 2 | | | | |
| Natrium Maisstärke n-Octenylsuccinat | | | | 0,5 | | 1 |
| Hydroxystearylhydroxystearat (Elfacos C26) | 2 | | 2 | | | |
| $C_{20-40}$ Alkylstearat (Kesterwachs K82) | 1 | 1 | | | | |
| $C_{16-38}$ Alkylhydroxystearoylstearat (Kesterwachs K80P) | | 2 | | 3 | | |
| Behenoxy Dimethicon (Abil Wax 2440) | | | 5 | | | |
| Polyisobuten (Rewopal PIB 1000) | 5 | | 5 | | | |
| Caprylic/Capric Triglycerid | 5 | 5 | 5 | 15 | 20 | 20 |
| Octyldodecanol | 5 | | 5 | 15 | | 15 |
| Mineralöl | 10 | | | 10 | | 20 |
| Butylen Glycol Caprylat/Caprat | | 10 | 10 | | 20 | 7 |
| $C_{12-15}$ Alkylbenzoat | 10 | 10 | 10 | 5 | 15 | |
| Dimethicon | | 2 | 3 | | | |
| Dicaprylyl Ether (Cetiol OE) | | | | 5 | | |

| | 1 | 2 | 3 | 4 | 5 | 6 |
|---|---|---|---|---|---|---|
| | W/O | W/O | W/O | O/W | O/W | O/W |
| Hydriertes Polyisobuten (Polysynlan) | 2 | | | | | |
| Methylbenzyliden Camphor | | 3 | | | 4 | |
| Octyltriazon | | 1 | | | 4 | |
| Dibenzoylmethan | | 2 | | | 2 | |
| Dioctyl Butamido Triazon (UVASORB HEB) | | 2 | | | | |
| Konservierung | 0,5 | 0,5 | 0,5 | 0,5 | 0,5 | 0,5 |
| Glycerin | 5 | 10 | 3 | 5 | 5 | 5 |
| Biosaccharid Gel (Fucogel 1000) | | | 5 | | | |
| Hyaluronsäure | | | | | 0,5 | |
| NaCl | 1 | | 1 | | | |
| $MgSO_4$ | | 0,5 | | | | |
| Phenylbenzimiazol Sulfonsäure | | 1 | | | 2 | |
| Carbomer (Carbopol 981) | | | | 0,1 | | |
| Xanthan Gum | | | | 0,3 | | |
| Cellulose Gum (Natrosol Plus 330 CS) | | | 0,1 | | | |
| NaOH 45%ige wässrige Lösung | | 0,3 | | 0,1 | 0,7 | |
| EDTA-Lösung | | 1 | | | 1 | |
| Wasser | ad 100 | ad 100 | ad 100 | ad 100 | ad 100 | ad 100 |

**Patentansprüche**

1. Pickering-Emulsionen, welche feindisperse Systeme vom Typ Wasser-in-Öl oder Öl-in-Wasser darstellen, enthaltend

    (1) eine Ölphase,
    (2) eine Wasserphase,
    (3) mindestens einen Typ mikrofeiner Partikel, die

        a) eine mittlere Partikelgröße von weniger als 200 nm haben, die
        b) sowohl hydrophile als auch lipophile Eigenschaften zeigen, welche also amphiphilen Charakter besitzen und sowohl in Wasser als auch in Öl dispergierbar sind und die
        c) gegebenenfalls oberflächlich beschichtet sind,

    (4) mindestens einen Typ nicht-amphiphiler Metalloxidpigmente und

(5) höchstens 0,5 Gew.-% eines oder mehrerer Emulgatoren.

2. Pickering-Emulsionen nach Anspruch 1, dadurch gekennzeichnet, daß sie emulgatorfrei sind.

3. Pickering-Emulsionen nach einem der vorhergehenden Ansprüche, dadurch gekennzeichnet, daß der Gehalt der amphiphilen Partikel zwischen 0,1 Gew.-% und 30 Gew.-% ist, bezogen auf das Gesamtgewicht der Zubereitungen.

4. Pickering-Emulsionen nach einem der vorhergehenden Ansprüche, dadurch gekennzeichnet, daß der Partikeldurchmesser der amphiphilen Partikel zwischen 5 nm und 100 nm liegt.

5. Pickering-Emulsionen nach einem der vorhergehenden Ansprüche, dadurch gekennzeichnet, daß die amphiphilen Partikel gewählt werden aus der Gruppe bestehend aus amphiphilen Metalloxiden, Bornitrid, mikrofeinen Polymerpartikeln und modifizierten Polysacchariden, wobei die Partikel sowohl einzeln als auch im Gemisch vorliegen können.

6. Pickering-Emulsionen nach einem der vorhergehenden Ansprüche, dadurch gekennzeichnet, daß die amphiphilen Partikel oberflächlich wasserabweisend behandelt sind, wobei der amphiphile Charakter der Partikel gebildet wird bzw. erhalten bleibt.

7. Pickering-Emulsionen nach einem der vorhergehenden Ansprüche, dadurch gekennzeichnet, daß die Gesamtmenge an nicht-amphiphilen Pigmenten in den fertigen kosmetischen oder dermatologischen Zubereitungen kleiner als 20 Gew.-%, bevorzugt zwischen 0,5 und 10 Gew.-% gewählt wird, bezogen auf das Gesamtgewicht der Zubereitungen.

8. Pickering-Emulsionen nach einem der vorhergehenden Ansprüche, dadurch gekennzeichnet, daß das oder die nicht-amphiphilen Pigmente gewählt werden aus der Gruppe der hydrophoben bzw. hydrophob beschichteten Metalloxide, insbesondere aus der Gruppe der hydrophoben bzw. hydrophob beschichteten Titandioxide.

9. Pickering-Emulsionen nach einem der vorhergehenden Ansprüche, dadurch gekennzeichnet, daß das oder die nicht-amphiphilen Pigmente gewählt werden aus der Gruppe der hydrophilen bzw. hydrophil beschichteten Metalloxide, insbesondere aus der Gruppe der hydrophilen bzw. hydrophil beschichteten Titandioxide.

10. Lichtschutzzubereitungen auf Basis von Pickering-Emulsionen nach einem der vorhergehenden Ansprüche.

11. Verwendung von mindestens einem Typ nicht-amphiphiler Metalloxidpigmente zur Erhöhung der UV-Schutzleistung kosmetischer oder dermatologischer Pickering-Emulsionen.